## Europäisches Patentamt

## European Patent Office

## Office européen des brevets

(19)

(11) Veröffentlichungsnummer: **0 176 954**

**A2**

(12) # EUROPÄISCHE PATENTANMELDUNG

(21) Anmeldenummer: **85112187.1**

(22) Anmeldetag: **26.09.85**

(51) Int. Cl.⁴: **G 01 N 27/12**
**G 01 N 33/00**

(30) Priorität: **29.09.84 DE 3435874**

(43) Veröffentlichungstag der Anmeldung:
**09.04.86 Patentblatt 86/15**

(84) Benannte Vertragsstaaten:
**AT BE CH DE FR GB IT LI LU NL SE**

(71) Anmelder: **Krauleidies, Horst**
**Possmoorweg 23**
**D-2000 Hamburg 60(DE)**

(72) Erfinder: **Krauleidies, Horst**
**Possmoorweg 23**
**D-2000 Hamburg 60(DE)**

(54) **Verfahren und Anordnung zur Überwachung der Fremdgaskonzentration in einem gasförmigen Trägermedium.**

(57) Verfahren und Geräte zur Fremdgaskonzentrationsermittlung in Abluft verwenden Sensoreinrichtungen, die in Abhängigkeit von Temperatur, Feuchte, Alterung und dergleichen ihr Betriebsverhalten sehr stark verändern, so daß in kurzen Intervallen eine Nachregelung der Geräte erfolgen muß, die im Betriebsfall aber in der Regel nicht möglich ist. Für den massenweisen Einsatz sind derartige Geräte und Verfahren im Zuge verstärkter Umweltschutzauflagen ungeeignet, da diese einerseits von fachlich geschulten Personen überwacht und eingestellt werden müssen, wobei andererseits sich aber derartige Verfahren im Betrieb praktisch als ungeeignet erwiesen haben. Um jedoch auch derartige Verfahren und Geräte durch Hilfskräfte bedienbar zu machen und ie aufgrund einfachen Aufbaus auch in großer Zahl einsetzen zu können, wird hier ein Verfahren zur Überwachung der Fremdgaskonzentration in einem gasförmigen Trägermedium vorgeschlagen, bei dem das in Abhängigkeit von der Fremdgasanreicherung im Trägermedium erzeugte Signalspektrum der Sensoreinrichtung fremdabgleichfrei positiv verstärkt wird und nachfolgend zur Erzeugung eines zeitabhängigen Signaldiagramms aufgezeichnet wird.

Fig. 1

EP 0 176 954 A2

Croydon Printing Company Ltd

Verfahren und Anordnung zur Überwachung der Fremdgaskonzentraktion in einem gasförmigen Trägermedium

## Beschreibung

Die Erfindung betrifft ein Verfahren sowie eine Anordnung zur Überwachung der Fremdgaskonzentration in einem
gasförmigen Trägermedium, insbesondere Abluft, mit einer mit dem Trägermedium in Kontakt stehenden elektrisch betriebenen Sensoreinrichtung, die in Abhängigkeit von Fremdgasanreicherung im Trägermedium Signale
liefert.

Im Zuge sich rapide verschärfender Umweltschutzmaßnahmen kommt der kontinuierlichen Überwachung der Abluft
bei industriellen Prozessen immer größere Bedeutung
zu. Um beispielsweise den Sättigungsgrad von Reinigungsfiltern, die die Abluft von Fremdgasen befreien
sollen, kontinuierlich zu überwachen, werden Gaskonzentrationsmeßgeräte eingesetzt, die, da es sich dabei um
hochgenaue Labormeßgeräte handelt, sehr kostspielig
sind und die Kosten von Filtereinrichtungen, beispielsweise Aktivkohlefiltern, um ein Vielfaches übersteigen. Derartige Gaskonzentrationsmeßgeräte eignen sich

0176954

deshalb wegen ihrer hohen Kosten ausschließlich für zu überwachende Großanlagen.

In der Regel behelfen sich die Betreiber von Filteranlagen damit, daß anhand von auf feste Zeitintervalle eingestellten Schaltuhren bei Erreichen der oberen Intervallgrenze die Filter unabhängig davon, ob sie die Grenze ihrer Filterfähigkeit erreicht haben oder nicht, einfach ausgewechselt werden. Auch dieses Verfahren ist äußerst nachteilig, da bei gesättigtem Filter ungehemmt das Fremdgas mit der Abluft entweichen kenn, wenn vor Erreichen der oberen Zeitschwelle das Filter schon gesättigt ist und somit eine den Auflagen widersprechende Umweltbelastung in Kauf genommen wird, oder daß bei geringer Fremdgaskonzentration in der zu filternden Abluft das Filter bei Erreichen der oberen Zeitschwelle entfernt wird, ohne daß das Filter tatsächlich schon die Grenzen seiner Filterfähigkeit erreicht hat, was einer kostspieligen Verschwendung von Filtermaterial entspricht oder erhöhte Kosten verursacht, weil vorzeitig eine Regenerierung erfolgt.

Schließlich sind mit dem Ziel einer Messung der tatsächlichen Fremdgaskonzentration im Trägermedium Geräte bekannt geworden, die Sensoren auf Halbleiterbasis verwenden, die einerseits die Fremdgaskonzentration feststellen sollen, damit die Filtersättigung präzise erkannt werden soll und die andererseits preisgünstig in der Herstellung und im Einsatz sein sollen, so daß sie sich auch für den massenweisen Einsatz in Filteranlagen eignen. Bei diesen bekannten Gaskonzentrationsmeßgeräten sind die Sensoren unmittelbar im im Fremdgas angereicherten Trägermedium angeordnet, beispielsweise unmittelbar im Abluftstrom. Diese Sensoren auf Halbleiterbasis zeigen physikalisch bedingte Veränderungen

- 3 -

0176954

ihrer Betriebsparameter in Abhängigkeit von Temperatur, Feuchte und Alterung, sowie sporadische Empfindlichkeitsänderungen, die regelungstechnisch praktisch nicht ausgleichbar sind. Äußerst nachteilig erwies es sich während des Betriebes dieser bekannten Geräte, daß infolge des temperatur-, feuchte- sowie alterungsbedingten Driftens der Betriebsparameter des Halbleitersensors eine Nachregelung auf einen bestimmten Null- bzw. sonstigen festen Betriebswert vorgenommen werden mußte, und zwar von mit meßtechnischen Problemen wenig vertrauten Personen, die in der Regel lediglich angelernte Überwachungspersonen sind. Das hat zur Folge, daß ebenfalls ein massenweiser Einsatz dieser bekannten Gaskonzentrationsmeßgeräte sich als nicht praktikabel herausgestellt hat, da ein physikalisch bedingtes Driften des Halbleitersensors kontinuierlich zur Erreichung vermeintlich verwendbarer Meßergebnisse nachgeregelt werden mußte, was personalbedingt für den massenweisen Einsatz zudem ein unüberwindbares Hindernis ist. Darüber hinaus ist die Nachregelung auch meßtechnisch bei kurzen Arbeitszyklen der Filter oft nicht durchführbar, was aber bei sporadischen Empfindlichkeitsänderungen des Halbleitersensors überhaupt nicht möglich ist, da das ein Mangel der Sensoreinrichtung selbst ist und durch Nachregeln auch nicht behoben werden kann.

Es ist Aufgabe der vorliegenden Erfindung, ein Verfahren sowie Anordnung in der eingangs genannten Art zu schaffen, bei denen der Einfluß umgebungsbedingter Betriebsparameteränderungen des Halbleitersensors stark verringert wird, das einfach durchführbar ist, wobei die Anordnung einfach im Aufbau ist und damit kostengünstig herstellbar ist und kostengünstig einsetzbar und frei von notwendigen Justiermaßnahmen während des Betriebs ist.

- 4 -                    0176954

Gelöst wird die Aufgabe gemäß der Erfindung dadurch, daß das Signalspektrum der Sensoreinrichtung fremdabgleichfrei positiv verstärkt und nachfolgend zur Erzeugung eines zeitabhängigen Signaldiagramms aufgezeichnet wird.

Die sporadischen Empfindlichkeitsänderungen und damit gegenenenfalls auch des Nullwertes, die den Überwachungsvorgang bisher so nachteilig beeinflußten, daß die ermittelten Ergebnisse zu einer völlig unbrauchbaren Aussage führten, werden bei dem hier beschriebenen Verfahren bewußt nicht unterdrückt bzw. es erfolgt keine Nachregelung durch die Bedienungsperson. Vielmehr sind diese Empfindlichkeitsänderungen oder die Änderungen anderer Betriebsparameter der Sensoreinrichtung Teil des während eines Betriebszyklus ermittelten Signaldiagramms, sind also bewußt sich.        _nd bewertbar gemacht.

Der Vorteil, der durch das Verfahren erzielt wird, liegt darin, daß die Bedienungsperson oder der Betreiber einer zu überwachenden Anlage in die Lage versetzt wird, daß aufgrund des nach dem Verfahren erzeugten Signaldiagramms nicht nur auf den Grad der Fremdgaskonzentration im Trägermedium geschlossen werden kann, sondern ebenfalls auf die Wertigkeit der Sensorsignale, das heißt das Betriebsverhalten der Sensoreinrichtung unter Einschluß seiner Betriebsparameteränderungen wird protokolliert, und damit bewertbar gemacht, so daß daraus wiederum auf die Wertigkeit des Signaldiagramms geschlossen werden kann.

Gemäß einer vorteilhaften Ausfüllungsform der Erfindung erfolgt die Aufzeichnung des durch die Sensorvorrichtung gelieferten Signalspektrums über eine Mehrzahl von

Überwachungszyklen, so daß durch die wiederholte Aufzeichnung des Fremdgasaufzeichnungsvorganges im Trägermedium eine noch bessere Beurteilung der Sensorsignale in bezug auf ihre Richtigkeit erfolgen kann, das heißt der Vertrauensgrad in die Bewertbarkeit der ermittelten Signale nimmt zu.

Ein weiterer Vorteil des Verfahrens ist, daß einerseits mit ihm keine Signale des von der Sensoreinrichtung gelieferten Signalspektrums verloren gehen, selbst bei extremen Betriebszuständen vorübergehender oder andauernder Art, und andererseits niemals Signale auftreten können, die unter einem bestimmten minimalen Wert oder oberhalb eines bestimmten maximalen Werts liegen.

Bei der Anordnung zur Ausführung des Verfahrens gemäß der Erfindung wird der Sensoreinrichtung über eine Entnahmeleitung mit Einlaßöffnung fremdgasangereichertes Trägermedium außerhalb der unmittelbaren Strömung der Entnahmeleitung liegend derart zugeführt, daß dieses die Sensoreinrichtung im wesentlichen strömungsfrei umhüllt.

Der Vorteil einer derartigen Anordnung der Sensoreinrichtung liegt darin, daß diese nicht unmittelbar in einem Abluftrohr bzw. Abluftkanal den Änderungen der Temperatur, dem Feuchtigkeitsgrad oder anderer sich ändernder Parameter des fremdgasangereicherten Trägermediums ausgesetzt ist, sondern daß es über eine mit einer Einlaßöffnung versehene Entnahmeleitung an der Sensoreinrichtung vorbeigeführt wird, so daß unmittelbare Beeinflussungen durch Änderungen durch Temperatur und Feuchte vermieden werden. Die Sensoreinrichtung ist dabei vorteilhafterweise so angeordnet, daß lediglich in

den Sensorraum diffundierendes bzw. eindringendes fremdgasangereichertes Trägermedium das eigentliche Sensorelement strömungsfrei umgibt, so daß realtiv stabile Reaktionen erzielt werden können, ohne daß das Sensorelement in seinem Betriebsverhalten gestört wird.

Da das Trägermedium neben seiner Anreicherung durch Fremdgase zusätzlich noch durch Staub oder Kondensat verunreinigt sein kann, Staub und Kondensat bei Niederschlag auf dem Sensorelement und in der Zuleitung wiederum zu nachteiligen Fehlreaktionen führt, ist gemäß einer vorteilhaften Ausführungsform in der Entnahmeleitung, ausgehend von der Einlaßöffnung, der Sensoreinrichtung nachfolgend eine Staub/Kondensatauffangeinrichtung angeordnet. Diese Staub/Kondensatauffangeinrichtung ist als Senke unterhalb der Sensoreinrichtung angebracht, so daß über einen in vertikaler Richtung unterhalb der Sensoreinrichtung angebrachten Einlaß Trägermedium einerseits staub- bzw. kondensatfrei zugeführt werden kann, da überwiegend der Staub bzw. das Kondensat nach unten in die unterhalb der Sensoreinrichtung und dem Einlaß angeordnete Staub/Kondensatauffangeinrichtung fällt, andererseits der Bedienungsperson durch aufgefangenen Staub/Kondensat angezeigt wird, daß das aufgezeichnete Signalspektrum von Staub/Kondensat beeinflußt worden ist, so daß dieses mit Fehlern behaftet sein kann.

Um zu gewährleisten, daß die Sensoreinrichtung in weiten Bereichen stets auf einfache Weise in einem bestimmten für die Überwachungsfunktion notwendigen Temperaturbereich gehalten werden kann, ist die Sensoreinrichtung auf einem einen Hohlraum aufweisenden metallischen Trägerkörper angeordnet, der den Einlaß zum Anschluß der Entnahmeleitung und einen Auslaß aufweist, wobei der Hohlraum mit dem Einlaß, dem Auslaß und der Sensoreinrichtung in Verbindung steht. Dieser metallische Trägerkörper, der topfförmig ausgebildet sein kann, schafft somit eine thermische Kopplung mit der Raumtemperatur, in der die Anordnung insgesamt steht und gleichzeitig eine thermische Entkopplung von der Temperatur des fremdgasangereicherten Trägermediums. Dadurch wird eine temperaturbedingte Drift des Sensorelements auf Halbleiterbasis weitgehend vermieden, so daß die Überwachungsfunktion mit hoher Zuverlässigkeit auf einfache Weise durchgeführt werden kann.

Um die Staub/Konücnsatablagerungen, die vom Trägermedium während des Meßvorganges mitgeführt werden, schnell ermitteln zu können, ist die am unteren Teil des Tragkörpers angeordnete Staub/Kondensatauffangeinrichtung vorteilhafterweise durchsichtig ausgebildet, so daß fortwährend eine einfache Kontrolle möglich ist und ggf. Maßnahmen zur Behebung dieses Nachteils schnell ergriffen werden können.

Um für die Kontrollperson schnell erkennbar zu machen, ob tatsächlich Trägermedium für den Meßvorgang der Sensoreinrichtung zugeführt wird, ist es von Vorteil, in der Entnahmeleitung, ausgehend von der Einlaßöffnung, der Sensoreinrichtung nachfolgend eine Druchströmanzeigevorrichtung vorzusehen. Dieser Durchströmanzeigeeinrichtung in Strömrichtung nachfolgend ist gemäß einer anderen vorteilhaften Ausführungsform eine Pumpe nachgeordnet, die dafür sorgt, daß kontinuierlich fremdgasangereichertes Trägermedium aus dem Ausstoßkanal bzw. -rohr über die Einlaßöffnung der Entnahmeleitung der Sensoreinrichtung zugeführt wird. Für die Ermittlung des Fremdanteils im Trägermedium ist es grundsätzlich nicht nötig, daß pro Zeitintervall immer bestimmte Mengen von Trägermedium am Sensor vorbeigeführt werden, jedoch wird durch die Pumpe sichergestellt, daß zumindest ausreichend Trägermedium zur Sensoreinrichtung gefördert wird. Es sind jedoch keine besonderen Maßnahmen zur Stabilisierung des Stroms des Trägermediums erforderlich.

Während des Betriebes der Anordnung können Betriebszustände bei der zu überwachenden Anlage auftreten, in denen eine Entnahme von fremdgasangereichertem Trägermedium nicht sinnvoll ist, beispielsweise bei sehr hohen Temperaturen bzw. sehr hohem Feuchtigkeitsgrad des Trägermediums, da dann die Signalwerte des Sensors nicht mehr fehlerfrei sind. Um in einem Betriebsprotokoll derart fehlerhafte Signalwerte bei derartigen Betriebszuständen zu unterdrücken, kann vorteilhafterweise in der Entnahmeleitung zwischen Einlaßöffnung und Sensoreinrichtung ein Dreiwegeventil vorgesehen werden, wobei der freie Anschluß des Ventils mit fremdgasfreiem Trägermedium beaufschlagbar ist, so daß bei Freigabe dieses Ventilweges zur Sensoreinrichtung eine Null-Phase ermittelt wird, was gleichzeitig einer Kontrolle eines unteren Signalwertebezugspunktes der Anlage insgesamt dienen kann.

Die Sensoreinrichtung, die als eigentliches Sensorelement ein heizbares Halbleiterelement aufweist, ist mit einer elektrischen Schaltung verbunden, die vorteilhafterweise im wesentlichen aus einer mit einem Verstärker zusammenwirkenden Brückenschaltung besteht, wobei die Sensoreinrichtung mit ihren Sensorklemmen mit dem mit dem Ausgang des Verstärkers verbundenen Widerstand der Brückenschaltung parallel geschaltet verbunden ist. Durch Änderung des Widerstandes des Sensorelementes in Abhängigkeit von der Beeinflussung durch im Trägermedium befindliche Fremdgasmoleküle wird der Widerstand der Brückenschaltung und damit das Ausgangssignal des Verstärkers geändert, so daß diese Änderung vorteilhafterweise auf einer der Schaltung nachfolgend angeordneten Anzeige der Fremdgaskonzentration angezeigt wird. Die Schaltung selbst kommt aufgrund der vorbeschriebenen Anordnung des Sensors vollständig ohne Kalibriereinrichtungen zur Nullpunktverschiebung aus, so daß die erfindgungsgemäße Anlage insgesamt von ungeschulten Personen sicher und einwandfrei bedient und überwacht werden kann. Schließlich ist es von Vorteil, um genaue Protokolle über einen beliebig langen Zeitraum erstellen zu können und eine einwandfreie Beurteilungsgrundlage für die sichere Funktion der Anordnung als auch ein einwandfreies Protokoll für den zu erfassenden Anteil an Fremdgas im Trägermedium zu haben, wenn die Schaltung zusätzlich mit einer Schreib/Druckeinrichtung verbunden ist. Diese Schreib/Druckeinrichtung kann nach entsprechender Anpassung an die Schaltung beliebiger, handelsüblicher Art sein.

Zur Kalibrierung der Anordnung sowie zu deren eigener Überwachung auf Funktionsfähigkeit ist es nötig, in geeigneten Zeitintervallen diese mit einem Kalibriergas zu beschicken, so daß Bezugsgrößen, beispielsweise in Form von Ausschlägen bestimmter Amplituden auf einer Schreib/Druckeinrichtung, ermittelt bzw. sichtbar gemacht werden können. Zu diesem Zweck wurden bisher vornehmlich undurchsichtige Behälter verwendet, in die mittels einer Mikroliterspritze eine bestimmte Menge eines Fremdmediums in ein durch den Behälter bestimmtes Volumen hineingegeben wurde, wo das Fremdmittel verdampfte und somit das im Behälter befindliche Trägermedium mit Fremdgasmolekülen anreicherte.

Da aber gerade für Kalibrierungsmaßnahmen der Anordnung insgesamt die Fremdgaskonzentration im Trägermittelvolumen hochgradig genau sein muß, eignen sich die bisher dafür verwendeten Behälter nur sehr schlecht, da von außen eine Überwachung über den tatsächlichen Verdampfungsgrad des Fremdmittels nicht möglich war. Insbesondere bei schwer, das heißt erst über einen längeren Zeitraum in den gasförmigen Zustand übergehende Fremdmittel, muß eine Kontrolle des tatsächlichen Übergangs in den gasförmigen Zustand kontinuierlich möglich sein und durchgeführt werden.

Es ist somit eine weitere Aufgabe der vorliegenden Erfindung, einen Behälter zur Erzeugung eines Kalibriergases zu schaffen, der in der vorangehend beschriebenen Anordnung verwendbar ist und der eine kontinuierliche Überwachung des Verdampfungsprozesses des Fremdmittels auf einfache Weise ermöglicht.

Gelöst wird die Aufgabe gemäß der Erfindung dadurch, daß der Behälter ein Behälterunterteil aufweist, das von einem metallischen Deckelteil verschlossen ist, wobei das Deckelteil eine erste Öffnung zum Anschluß an die Entnahmeleitung und zweite Öffnung zum Einbringen eines Kalibriermittels aufweist, das auf einem am Deckel innen befestigten, in den Behälter hineinragenden Träger ablegbar ist.

Neben der einfachen Handhabbarkeit, die Mikroliterspritze wird mit ihrem Nadelteil bequem durch die zweite Öffnung eingeführt, so daß bei Berührung der Nadelspitze mit dem Träger dort das Fremdmittel auflegbar ist, bietet der Behälter die Möglichkeit, auf einfache Art nachfolgend den Übergang des Fremdmittels in den gasförmigen Zustand fortlaufend zu überwachen.

Das Behälterunterteil besteht vorzugsweise aus Glas, so daß vorkonfektionierte Glasgefäße sich ohne weiteres für den hier vorgeschlagenen Einsatz eignen. Gemäß einer vorteilhaften Ausführungsform besteht das Deckelteil sowie der Träger aus nichtrostendem metallischem Werkstoff, beispielsweise Stahl, so daß der Nachteil ständig gasender und somit zu Fehlmessungen führender Kunststoffteile vermieden wird.

Um sicherzustellen, daß das mit der Mikroliterspritze in den Behälter eingebrachte Fremdmittel auch vollständig, das heißt in vorbestimmter Menge eingebracht wird, ist der Träger vorteilhafterweise elastisch ausgebildet, so daß die Spitze der Mikroliterspritze zunächst in Anlage an den Träger gebracht werden kann, der nachfolgend zusammen mit der Spritze sich elastisch verformend heruntergedrückt wird. Dadurch wird ein einwandfreier Kontakt zwischen Träger und Spritzenspitze sichergestellt, so daß tatsächlich sämtliches Fremdmittel auf dem Träger im Behälterinneren eingebracht wird. Die Benetzungskräfte zwischen Fremdmittel und Träger halten nachfolgend das Fremdmittel auf ihm, bis es vollständig in den gasförmigen Zustand übergegangen ist.

Nachfolgend erfolgt durch Molekularbewegung zwischen dem Trägermittel und dem in den gasförmigen Zustand übergegangenen Fremdmittel eine Durchmischung zwischen beiden, wobei der Austritt aus dem Behälter zur nachfolgenden Kalibrierung der Anordnung das Deckelteil des Behälters vorteilhafterweise an seiner ersten Öffnung mit einem in den Behälter hineinragenden Rohr versehen ist, das an seinem vom Deckelteil wegweisenden Ende eine Öffnung aufweist, in die das fremdgasversetzte Trägermedium, das heiß das Kalibriergas, vollständig durchmischt eindringen und von dort über die Entnahmeleitung auf die Sensoreinrichtung gegeben werden kann.

Die Erfindung wird nun unter Bezugnahme auf die nachfolgenden schematischen Zeichnungen anhand eines Ausführungsbeispieles eingehend beschrieben. Darin zeigen:

Fig. 1 einen schematischen Aufbau einer Anordnung insgesamt im Zusammenwirken mit einer Filteranlage,

Fig. 2 einen schematischen Aufbau des Trägerkörpers mit
eingesetzter Sensoreinrichtung im Schnitt,

Fig. 3 in vergrößerter schematischer Darstellung eine
Sensoreinrichtung im Schnitt,

Fig. 4 eine Schaltung im Zusammenwirken mit der Sensoreinrichtung sowie einer Einrichtung zur Anzeige
der der Fremdgaskonzentration entsprechenden
elektrischen Signalgröße,

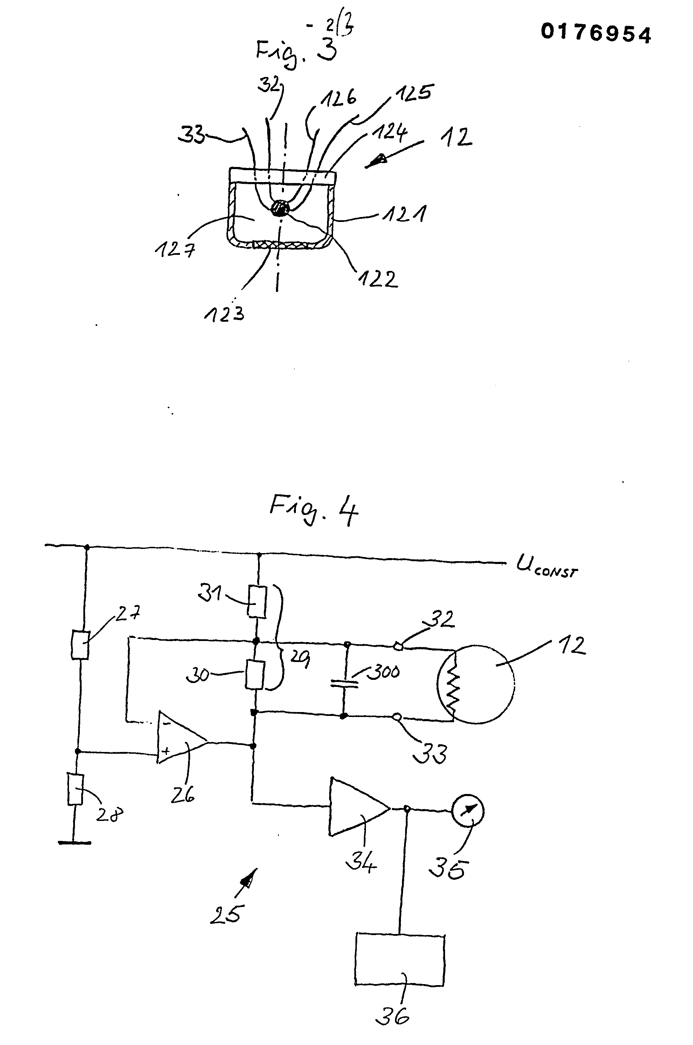

Fig. 5 in schematischer Darstellung einen Behälter zur
Erzeugung eines Kalibriergases im Schnitt und

Fig. 6 den Verlauf eines mit der Anordnung ermittelten
typischen Signalspektrums unter Einschluß dreier
Kalibrierungsvorgänge und einem Fremdgasanteil
im Trägermittelvolumen von 0,5, 1, und 2.

Die Anordnung besteht gemäß dem hier beschriebenen Ausführungsbeispiel im wesentlichen aus einer Sensoreinrichtung 12, die über eine Entnahmeleitung 13 mit Einlaßöffnung 14 mit einem Rohr bzw. Kanal 11 in Verbindung steht, in dem das fremdgas-angereicherte Trägermedium, beispielsweise Abluft, nach Passieren einer Filtereinrichtung abgeführt wird. Die Sensoreinrichtung 12 steht wiederum mit einer elektrischen Schaltung 25 in Verbindung, die ihrerseits Werte für eine Anzeigeeinrichtung 35 bzw. eine ebenfalls damit verbundene Schreib/Druckeinrichtung 36 liefert.

Nach Passieren der Sensoreinrichtung 12 wird das Trägermedium über eine Förderleitung 130 zu einer Durchströmanzeigeeinrichtung 21 geführt, an der die koninuierliche Förderung des fremdgasangereicherten Trägermediums beobachtet werden kann. Bewirkt wird die kontinuierliche Förderung des Trägermediums aus dem Rohr 11 über die Einlaßöffnung 14, die Entnahmeleitung 13 an der Sensoreinrichtung 12 vorbei und durch die Durchströmanzeigeeinrichtung 21 hindurch durch eine der Durchströmanzeigeeinrichtung 21 in der Förderleitung 130 nachgeordnete Pumpe 22.

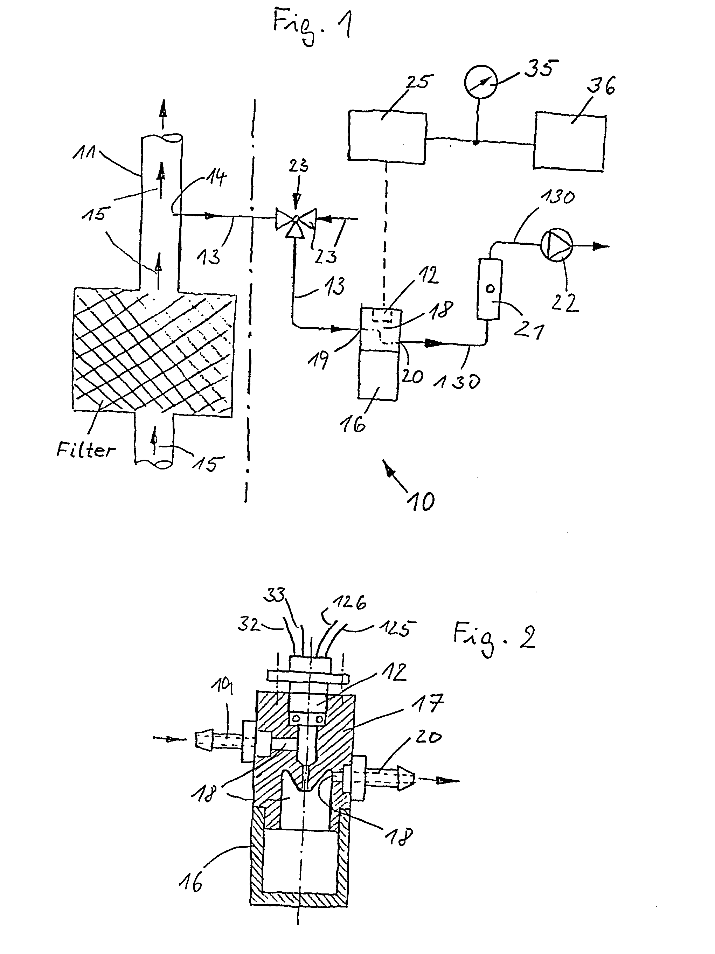

In die Entnahmeleitung 13, die mehrere Meter lang sein kann, so daß die Anordnung 10 in einem gesonderten Raum abgesetzt von der zu überwachenden Filtereinrichtung angeordnet sein kann, was durch die in Fig. 1 dargestellte strichpunktierte Linie angedeutet ist, ist ein Dreiwegeventil 23 angeordnet. Das Dreiwegeventil 23 in der Entnahmeleitung 13 ermöglicht es, daß neben dem zu messenden Trägermedium 15 über den freien Anschuß 24 Trägermedium zur Sensoreinrichtung 12 gelangen kann, sogenanntes Null-Medium, das frei von Fremdgasen ist und ermöglicht, daß ein unterer Bezugswert für die Messung insgesamt geschaffen werden kann.

Die Sensoreinrichtung 12 ist am oberen Ende eines metallischen Trägerkörpers 17 so angeordnet, daß das fremdgasangereicherte Trägermedium in einem im Trägerkörper 17 ausgebildeten Hohlraum 18 mit der fremdgasempfindlichen Stelle der Sensoreinrichtung 12 in Kontakt treten kann. Der Hohlraum 18 weist einen Einlaß 19 zum Anschluß der Entnahmeleitung 13 und einen Auslaß 20 zum Anschluß der Förderleitung 130 auf. Der Einlaß 19 ist in vertikaler Richtung unterhalb der Sensoreinrichtung 12 angebracht und in vertikaler Richtung darunter der Auslaß. Der Hohlraum 18 wird in vertikaler Richtung von unten von einer Staub/Kondensatauffangeinrichtung 16 abgeschlossen, die durchsichtig ausgebildet sein kann. Der Hohlraum 18 ist somit so ausgestaltet, daß über ihn die Sensoreinrichtung 12, der Einlaß 19, der Auslaß 20 und die Staub/Kondensatauffangeinrichtung 16 miteinander in Verbindung stehen, wie es in Fig. 2 dargestellt ist.

Die Sensoreinrichtung 12 besteht, wie in Fig. 3 dargestellt, aus dem eigentlichen Sensorelement 122, das in einem Sensorgehäuse, bestehend aus Sensorunterteil 121 und Aufnahmekörper 124 angeordnet ist. Der Aufnahmekörper 124, der in der Regel plattenförmig ausgebildet ist, bildet einen äußeren Verschluß, so daß die Sensoreinrichtung 12 insgesamt in den Trägerkörper 17 eingesetzt, dessen Hohlraum 18 oben hermetisch verschließt. Das topfförmige Sensorunterteil 121 weist eine mit Drahtgaze verschlossene Öffnung 123 auf. Da der Sensorkörper somit nur eine einzige Öffnung 123 aufweist, kann sich beim Eintritt von fremdgasangereichertem Trägermedium in den Hohlraum 127 keine Strömung um das eigentliche Sensorelement 122 ausbilden. Das Sensorelement 122 weist zwei Anschlußklemmen 32 und 33 auf, die mit der elektrischen Schaltung 25 in Verbindung stehen. Darüber hinaus weist es zwei Heizungsanschlußklemmen 125 und 126 auf, die mit einer geeigneten elektrischen Heizstromquelle verbunden werden.

Die in Fig. 4 dargestellte elektrische Schaltung 25 besteht im wesentlichen aus einem Verstärker 26, der beispielsweise ein Operationsverstärker sein kann, dessen positiver Eingang über einen Spannungsteiler, bestehend aus den Widerständen 27 und 28, auf ein geeignetes Potential eingestellt ist. Der Ausgang des Verstärkers ist über eine Brückenschaltung 29, bestehend aus den Widerständen 30 und 31, mit der Versorgungsspannung der Meßschaltung 25 verbunden. Die Widerstände 30 und 31 sind dabei in Reihe geschaltet. Die Verbindung zwischen den beiden Widerständen 30 und 31 ist wiederum mit dem negativen Eingang des Verstärkers 26 verbunden. Parallel zum Widerstand 30 ist die Sensoreinrichtung 12 mit ihren Klemmen 32 und 33 geschaltet sowie ein Kondensator 300, mit dem der Rauschpegel der Signale der Sensoreinrichtung 12 vermindert wird. Darüber hinaus ist der Ausgang des Verstärkers 26 mit einem Eingang eines weiteren Verstärkers 34 verbunden, der wiederum ausgangsseitig mit der Anzeigeeinrichtung 35 bzw. einer Schreib/Druckeinrichtung 36 in Verbindung steht.

Während des Überwachungsbetriebes der Anordnung 10 wird über die in das Rohr oder den Kanal 1 hineinragende Einlaßöffnung 14 der Entnahmeleitung 13 fremdgasangereichertes Trägermedium 15 fortlaufend mit Hilfe der Pumpe 22 entnommen. Dabei ist das Dreiwegeventil 23 so geschaltet, daß das Trägermedium unmittelbar in den Bereich der Sensoreinrichtung 12 gelangen kann. Zweckmäßigerweise ist die Entnahmeleitung 13 vertikal fallend angeordnet, so daß Schwebeteile im Trägermedium nach unten fallen und schließlich in die Staub/Kondensatauffangeinrichtung 16 unterhalb der Sensoreinrichtung 12 gelangen können und sichtbar für weitere Maßnahmen werden. Beim Eintritt in den Hohlraum 18 über den Einlaß 19 hin zum Auslaß 20 des Trägerkörpers 17 dringt ein bestimmter Teil des fremdgasangereicherten Trägermediums in den Innenraum 127 des Sensorelements ein, und zwar durch dessen Öffnung 123, ohne jedoch eine signalverzerrende Strömung um das Sensorelement 122 herum zu erzeugen. Temperatureinflüsse, die die Reaktion des Sensorelements 122 beträchtlich stören können, werden dadurch auf ein Minimum vermindert, daß die Sensoreinrichtung 12 in den metallischen Trägerkörper 17 eingebettet ist, der wiederum auf der konstanten Temperatur des Raumes gehalten wird, in der sich die Anordnung 10 insgesamt befindet. In Abhängigkeit vom Grad der Fremdgaskonzentration im Trägermedium wird sich der Widerstand des Sensorelements 122 verändern, was zu einer Veränderung des Widerstandes der Brückenschaltung 29 der elektrischen Meßschaltung führen wird. Infolgedessen verändert sich der Ausgangspegel des Verstärkers 26, was wiederum über die Anzeigeeinrichtung 35 bzw. die Schreib/Druckeinrichtung als Zu- bzw. Abnahme der Fremdkörpergaskonzentration ersichtlich ist.

Darüber hinaus gestattet die Schaltung 25 eine einfache Überprüfung ihrer eigenen Funktionsfähigkeit und zwar auf folgende Weise: durch Abklemmen der Sensoreinrichtung 12 von Widerstand 30, was einem unendlichen Sensorwiderstand entspricht, muß das mit der Anordnung 10 ermittelte Signaldiagramm einen Signalwert entsprechend 0% anzeigen, wobei die Widerstände 30, 31 der Brücke 29 gleich groß sind, beispielsweise 10 kOhm, 5 kOhm oder 1 kOhm, da der Sensorwiderstand bei unterschiedlichen Sensoreinrichtungen 12 je nach Typ unterschiedlich sein kann.

Wird die Sensoreinrichtung 12 an ihren Klemmen 32, 33 kurzgeschlossen, was einem Widerstand von 0 Ohm entspricht, muß das ermittelte Signaldiagramm einen Signalwert entsprechend 100% anzeigen.

Wird ein Referenzwiderstand entsprechend dem Widerstandswert von Widerstand 30 anstelle der Sensoreinrichtung parallel zum Widerstand 30 gelegt, muß das ermittelte Signalspektrum einen Signalwert von 50% anzeigen.

Durch diese Art des Aufbaus der Anordnung 10, insbesondere der Schaltung 25 ist es möglich, den bei bisherigen Gaskonzentrationsmeßgeräten notwendigen Null-Abgleich vollständig auszuschließen, der in Folge der von außen bedingten Änderungen der Betriebsparameter der Sensoreinrichtungen bisher notwendig ist. Selbst bei der Anordnung 10 nicht vollständig vermeidbare Änderungen der Betriebsparameter während des Einsatzes erfordern kein manuelles Nachregeln, da die Schreib/Druckeinrichtung dem Betreiber der Anlage nach Protokollierung mehrerer Betriebszyklen ein gutes Beurteilungskriterium für die Wertigkeit des Signalspektrums bietet. Nachregelung, wie bei den bekannten Geräten, würde im vorliegenden Fall vielmehr zu einem fehlinterpretierbaren Signalspektrum führen, da ein nachgeregelter Null-Punkt bzw. unterer Bezugswert die Signale und damit das Signalspektrum insgesamt beeinflussen muß.

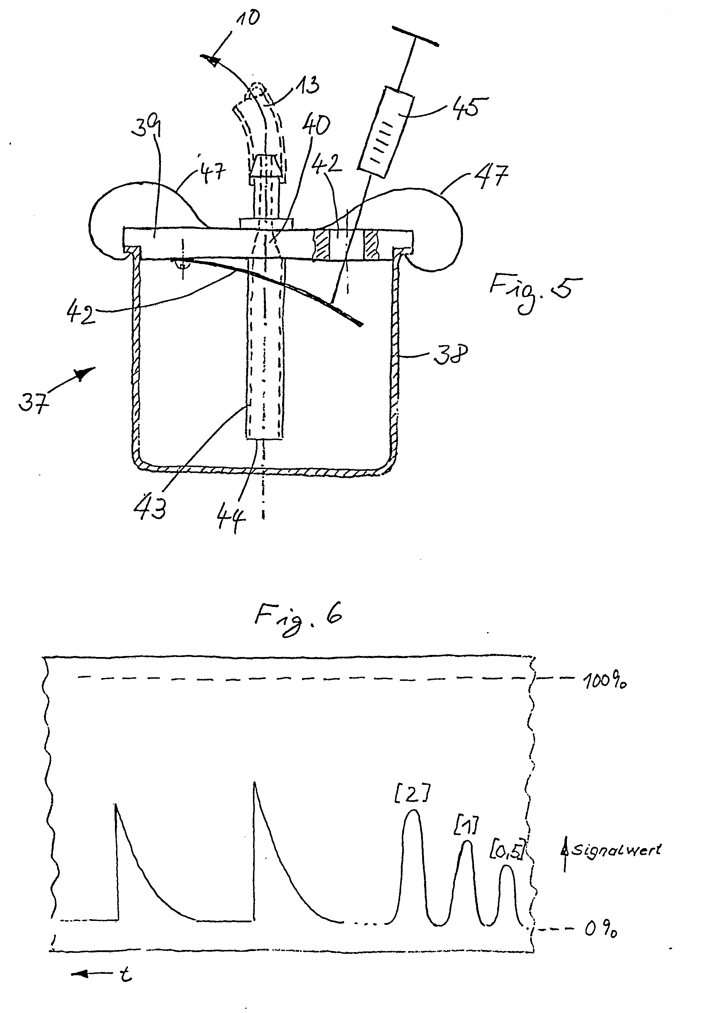

Das in Fig. 6 dargestellte beispielhafte Protokoll mehrerer Betriebszyklen, das zwischen 0% und 100% schwankende Werte umfassen kann, weist neben den beispielhaft aufgezeigten beiden ansteigenden und abrupt abfallenden Kurven zweier Betriebszyklen noch drei peakartige Kurven auf, die durch Beschicken der Anordnung 10 mit einem Kalibriergas gewonnen worden sind.

Das Kalibriergas, das ebenfalls über die Einlaßöffnung 14 in die Anordnung 10 gelangen kann, weist bei diesen drei schematisch dargestellten Kalibrierpeaks unterschiedliche Fremdgasanteile auf, die genau vorbestimmt sind und somit als Bezugsgrößen für die ermittelten Signale insgesamt herangezogen werden können. Darüber hinaus kann durch unterschiedlichen Fremdgasanteil im Kalibriergas ebenfalls ein Aufschluß über die Linearität der Anordnung insgesamt gewonnen werden.

Mit der Schaltung 25 können darüber hinaus Einrichtungen gesteuert werden, die bei Erreichen einer bestimmten Schaltschwelle des Verstärkers 26 entsprechend einer bestimmten Fremdgaskonzentration im Trägermedium angesteuert werden, beispielsweise Alarmgeber oder Schalteinrichtungen, die die zu überwachende Anlage insgesamt ausschalten.

Da für die genaue Kalibrierung der Anordnung 10 aber auch anderer, mit einem Kalibriergas zu kalibrierende Anlagen, Kalibriergasvolumen präzise hergestellt werden müssen, wird ein Behälter 37 verwendet, der aus einem durchsichtigen Behälterunterteil 38 und einem beispielsweise aus nichtrostendem Stahl hergestellten Deckelteil 39 besteht, wobei großvolumige Behälter 37 lediglich ein Sichtfenster aufweisen können, während das übrige Behälterteil 38 aus korosionsbeständigem Metall, beispielsweise aus nichtrostendem Stahl, bestehen kann. Das Deckelteil 39 weist einen Ansatz auf, mit dem es auf dem Behälterunterteil 38 an dessen Öffnung aufliegt. Das Deckelteil weist eine Öffnung 40 auf, auf der außen ein Rohransatz befestigt ist, auf dem die flexible Entnahmeleitung 13 der Anordnung 10 zu Kalibrierzwecken aufgesteckt werden kann. Darüber hinaus weist das Deckelteil 39 eine zweite Öffnung 41 auf, durch die die Nadel einer Mikroliterspritze zwecks Einbringung eines Fremdmittels in den Innenraum des Behälters 37 hindurchgesteckt werden kann. Am in den Behälterinnenraum weisenden Unterteil des Deckels 39 ist ein Träger 42 angebracht, der ebenfalls aus nichtrostendem Stahl in Form eines elastisch verformbaren Bleches ausgebildet ist. Ein Rohr 43 erstreckt sich von der ersten Öffnung 40 in den Innenraum des Behälters 37 bis in den Bereich des Behälterbodens, wobei das Rohr an seiner zum Behälterboden gerichteten Seite eine Öffnung 44 aufweist.

Nachdem der Behälterinnenraum mit bekanntem Volumen mit einem Trägermedium gefüllt worden ist, beispielsweise mit Luft, wird das Deckelteil 49 auf das Behälterunterteil 38 aufgesetzt, wobei beide Teile durch eine Klammer 47 zusammengehalten werden. Dann wird eine Mikroliterspritze oder ein anderes geeignetes Injetionsgerät durch die zweite Öffnung hindurchgeführt, wobei die Spitze des Injektionsgerätes auf den Träger 42 gedrückt wird. Nachfolgend wird der Träger durch weiteren Druck auf das Injektionsgerät nach unten gedrückt, so daß sichergestellt wird, daß sämtliches Fremdmittel, das im Behälter 37 nachfolgend in den gasförmigen Zustand übergehen soll, tatsächlich auf den Träger 42 gelangt, so daß bei Betätigung der Injektionseinrichtung kein Anteil des Fremdmittels an der Nadelspitze verbleibt. Nachfolgend wird die Injektionseinrichtung wieder aus der Öffnung herausgezogen.

Durch die durchsichtige Ausbildung des Behälterunterteils 38 kann die Bedienungsperson fortlaufend den Übergang in den gasförmigen Zustand überprüfen, so daß eine tatsächliche Gewähr für die Genauigkeit des so erzeugten Kalibriergases gegeben werden kann. Nach voll-

- 20 -  0176954

ständigem Übergang in den gasförmigen Zustand und anschließender Durchmischung mit dem Trägermedium infolge der Molekularbewegung kann das fertige Kalibriergas über die erste Öffnung 40 auf die Entnahmeleitung 13 der Anlage 10 gegeben werden, so daß eine präzise Kalibrierung der Anordnung 10 ermöglicht werden kann, wie es in Fig. 6 durch die drei Kalibrierkurven im dort dargestellten schematischen Protokoll ersichtlich ist.

Die Öffnung des Behälterunterteils 38 ist groß ausgebildet, beispielsweise entsprechend seinen Innenabmessungen, so daß für die Herstellung eines neuen Kalibriergases das andere, oder Reste von ihm, die sich noch im Innenraum befinden, einfach ausgegossen werden können. Weitere Hilfsmittel sind nicht nötig. Durch die einfache Handhabbarkeit sind auch fachlich nicht geschulte Bedienungspersonen in der Lage, von Zeit zu Zeit, je nach Erfordernis, eine Überprüfung des Sensors vorzunehmen.

## Bezugszeichenliste

| | | | |
|---|---|---|---|
| 10. | Anordnung | 300. | Kondensator |
| 11. | Rohr/Kanal zur Führung des Trägermediums | 31. | Widerstand |
| 12. | Sensoreneinrichtung | 32. | Klemme des Sensors |
| 121. | Sensorunterteil | 33. | Klemme des Sensors |
| 122. | Sensorelement | 34. | Verstärker |
| 123. | mit Drahtgaze verschlossene Öffnung | 35. | Anzeigeeinrichtung |
| 124. | Aufnahmekörper | 36. | Schreib/Druckeinrichtung |
| 125. | Heizungsanschlußklemme | 37. | Behälter |
| 126. | Heizungsanschlußklemme | 38. | Behälterunterteil |
| 127. | Innenraum | 39. | Deckelteil |
| 13. | Entnahmeleitung | 40. | erste Öffnung |
| 130. | Förderleitung | 41. | zweite Öffnung |
| 14. | Einlaßöffnung | 42. | Träger |
| 15. | Trägermedium | 43. | Rohr |
| 16. | Staub/Kondensatauffangeinrichtung | 44. | Öffnung |
| 17. | Trägerkörper | 45. | Mikroliterspritze |
| 18. | Hohlraum | 47. | Kammer |
| 19. | Einlaß | | |
| 20. | Auslaß | | |
| 21. | Durchströmanzeigeeinrichtung | | |
| 22. | Pumpe | | |
| 23. | Dreiwegeventil | | |
| 24. | freier Anschluß | | |
| 25. | elektrische Schaltung | | |
| 26. | Verstärker | | |
| 27. | Widerstand | | |
| 28. | Widerstand | | |
| 29. | Brückenschaltung | | |
| 30. | Widerstand | | |

Patentansprüche

1. Verfahren zur Überwachung der Fremdgaskonzentration in einem gasförmigen Trägermedium mit einer mit dem Trägermedium in Kontakt stehenden elektrisch betriebenen Sensoreinrichtung, die in Abhängigkeit von einer Fremdgasanreicherung im Trägermedium Signale liefert, dadurch gekennzeichnet, daß das Signalspektrum der Sensoreinrichtung fremdabgleichfrei positiv verstärkt und nachfolgend zur Erzeugung eines zeitabhängigen Signaldiagramms aufgezeichnet wird.

2. Verfahren nach Anspruch 1, dadurch gekennzeichnet, daß die Aufzeichnung des durch die Sensoreinrichtung gelieferten Signalspektrums über eine Mehrzahl von Überwachungszyklen erfolgt.

3. Verfahren nach einem oder beiden der Ansprüche 1 oder 2, dadurch gekennzeichnet, daß die Sensoreinrichtung zur Erzeugung eines Kalibrierspektrums mit einem eine unbestimmte Fremdgaskonzentration in einem vorbestimmten Trägermediumvolumen aufweisenden Kalibriergas beaufschlagt wird.

4. Anordnung zur Überwachung der Fremdgaskonzentration in einem gasförmigen Trägermedium, insbesondere Abluft, mit einer mit dem Trägermedium in Kontakt stehenden elektrisch betriebenen Sensoreinrichtung, zur Ausführung des Verfahrens nach Anspruch 1, dadurch gekennzeichnet, daß der Sensoreinrichtung (12) über eine Entnahmeleitung (13) mit Einlaßöffnung (14) fremdgasangereichertes Trägermedium (15) außerhalb der unmittelbaren Strömung der Entnahmeleitung (13) liegend derart zugeführt wird, daß dieses die Sonsorenrichtung (12) im wesentlichen strömungsfrei umhüllt.

5. Anordnung nach Anspruch 4, dadurch gekennzeichnet, daß in der Entnahmeleitung (13), ausgehend von der Einlaßöffnung (14), der Sensoreinrichtung (12) nachfolgend eine Staub/Kondensatauffangeinrichtung (16) angeordnet ist.

6. Anordnung nach einem oder beiden der Ansprüche 4 oder 5, dadurch gekennzeichnet, daß die Sensoreinrichtung (12) auf einem einen Hohlraum (18) aufweisenden metallischen Trägerkörper (17) angeordnet ist, der einen Einlaß (19) zum Anschluß der Entnahmeleitung (13) und einen Auslaß (20) aufweist, wobei der Hohlraum (18) mit dem Einlaß (19), dem Auslaß (20) und der Sensoreinrichtung (112) in Verbindung steht.

7. Anordnung nach Anspruch 6, dadurch gekennzeichnet, daß die Staub/Kondensatauffangeinrichtung (16) am unteren Teil des Trägerkörpers (17) angeordnet ist.

8. Anordnung nach einem oder mehreren der Ansprüche 4-7, dadurch gekennzeichnet, daß die Staub/Kondensatauffangeinrichtung (16) durchsichtig ausgebildet ist.

9. Anordnung nach einem oder mehreren der Ansprüche 4-8, dadurch gekennzeichnet, daß in der Entnahmeleitung (13), ausgehend von der Einlaßöffnung (14), der Sensoreneinrichtung (12) nachfolgend eine Durchströmanzeigeeinrichtung (21) angeordnet ist.

10. Anordnung nach einem oder mehreren der Ansprüche 4-9, dadurch gekennzeichnet, daß in der Entnahmeleitung (13), ausgehend von der Einlaßöffnung (14), der Sensoreinrichtung (12) nachfolgend eine Pumpe (22) angeordnet ist.

11. Anordnung nach einem oder mehreren der Ansprüche 4-10, dadurch gekennzeichnet, daß in der Entnahmeleitung (13) zwischen Einlaßöffnung (14) und Sensoreinrichtung (12) ein Dreiwegeventil (23) angeordnet ist, wobei der eine freie Anschluß (24) des Ventils (23) mit fremdgasfreiem Trägermedium beaufschlagbar ist.

12. Anordnung nach einem oder mehreren der Ansprüche 4-11, dadurch gekennzeichnet, daß die Sensoreinrichtung (12) mit einer elektrischen Schaltung (25) verbunden ist.

13. Anordnung nach Anspruch 12, dadurch gekennzeichnet, daß die Schaltung (25) im wesentlichen aus einer mit einem Verstärker (26) zusammenwirkenden Brückenschaltung (29) besteht, wobei die Sensoreinrichtung (12) mit ihren Sensorklemmen (32, 33) mit dem mit dem Ausgang des Verstärkers (26) verbundenen Widerstand (30) der Brückenschaltung (29) parallel geschaltet verbunden ist.

14. Anordnung nach Anspruch 13, dadurch gekennzeichnet, daß dem Widerstand (30) der Brückenschaltung (29) ein Kondensator (300) parallel geschaltet ist.

- 25 -

15. Anordnung nach einem oder mehreren der Ansprüche 12 bis 14, dadurch gekennzeichnet, daß die Schaltung (25) mit einer Einrichtung (35) zur Anzeige der elektrischen Signalgröße verbunden ist.

16. Anordnung nach einem oder mehreren der Ansprüche 12 bis 15, dadurch gekennzeichnet, daß die Schaltung (25) mit einer Schreib/Druckereinrichtung (36) verbunden ist.

17. Behälter zur Erzeugung eines Kalibriergases zur Verwendung in der Anordnung nach einem oder mehreren der Ansprüche 4-16, dadurch gekennzeichnet, daß der Behälter (37) ein Behälterunterteil (38) aufweist, das von einem metallischen Deckelteil (39) verschlossen ist, wobei das Deckelteil (39) eine erste Öffnung (40) zum Anschluß an die Entnahmeleitung (13) und eine zweite Öffnung (41) zum Einbringen eines Kalibriermittels aufweist, das auf einem am Deckel (39) innen befestigten, in den Behälter (37) hineinragenden Träger (42) ablegbar ist.

18. Behälter nach Anspruch 17, dadurch gekennzeichnet, daß das Behälterunterteil (38) wenigstens teilweise undurchsichtig ist.

19. Behälter nach einem oder beiden der Ansprüche 17 oder 18, dadurch gekennzeichnet, daß das Deckelteil (39) aus nichtrostendem Stahl besteht.

20. Behälter nach einem oder mehreren der Ansprüche 17-19, dadurch gekennzeichnet, daß der Träger (42) elastisch ist und aus nichtrostendem Stahl besteht.

21. Behälter nach einem oder mehreren der Ansprüche 17 bis 20, dadurch gekennzeichnet, daß das Deckelteil (39) an seiner ersten Öffnung (40) mit einem in den Behälter (37) hineinragenden Rohr (43) versehen ist, das an seinem vom Deckelteil (39) wegweisenden Ende eine Öffnung (44) aufweist.

Fig. 1

Filter

10

Fig. 2

0176954

**Fig. 3**

**Fig. 4**

$U_{CONST}$

Fig. 5

Fig. 6